Europäisches Patentamt

European Patent Office

Office européen des brevets

(11) Publication number: **0 474 218 A1**

## EUROPEAN PATENT APPLICATION

(21) Application number: **91114947.4**

(22) Date of filing: **04.09.91**

(51) Int. Cl.5: **A61M 5/315**

(30) Priority: **04.09.90 US 577116**
**14.09.90 US 582353**

(43) Date of publication of application:
**11.03.92 Bulletin 92/11**

(84) Designated Contracting States:
**BE DE ES FR GB IT NL**

(71) Applicant: **MENTOR CORPORATION**
**1499 West River Road North**
**Minneapolis, Minnesota 55411(US)**

(72) Inventor: **Nowakowski, Karol**
**5476 Landmark Circle**
**Moundsview, Minnesota 55112(US)**

(74) Representative: **Schwan, Gerhard, Dipl.-Ing.**
**Elfenstrasse 32**
**W-8000 München 83(DE)**

(54) Medical paste syringe, filling method and paste injecting device.

(57) A disposable syringe cylinder (60) and a medical paste injector (110) into which the cylinder (60) may be easily inserted. The cylinder (60) is prefilled by the supplier with a medical paste. A flexible piston (78) having a flat proximal surface is inserted by the supplier into the proximal end of the cylinder (60). The piston (78) serves as a seal between the paste and the outer environment. Proximal-to-distal force applied on the piston (78) by the injector (110) propels paste from the cylinder (60). The direction of travel of the flexible piston (78) is only proximal-to-distal, and the disposable syringe cylinder (60) is discarded when empty. The injector (110) includes an injector pump which is manually actuated by a pistol grip. A highly precise ratchet mechanism, along with an accurately positioned stop on the travel of the pistol grip, permits the attending medical personnel to deliver the exact volume of paste required.

FIG. 5

Rank Xerox (UK) Business Services

EP 0 474 218 A1

## BACKGROUND OF THE INVENTION

### 1. Field of the Invention

The present invention relates generally to medical devices and procedures, and more particularly, relates to packaging techniques for medical paste and to devices for injecting such paste.

### 2. Description of the Prior Art

It has been known for some time to inject a paste or semi-plastic material to reinforce a muscle, such as the urethral sphincter. In this procedure, a catheter is advanced in the urethra to a position adjacent the sphincter muscle. A desired amount of a paste such as PTFE paste is pumped under high pressure to the treatment area near the distal end of the catheter. The outside dimensions of the catheter are established by the necessity to traverse the urethra.

Medical injection pumps suitable to this task have been available for some time. US-A-4 551 135 describes an injection syringe which provides for the mixing of two different materials at the time of injection.

A pistol grip style injection syringe is discussed in US-A- 4,276,878. This particular pump employs a flexible piston rod with a series of ball-shaped pistons along its length.

US-A-4 820 287 shows a thumb operated pump for medical paste. The emphasis of the disclosure is a safety feature for pressure control.

A thumb-operated Teflon syringe is commercially available from the German Company of Karl Storz as Model 27200 A. This device uses a disposable syringe cylinder to hold the medical paste during the dispensing operation. The disposable syringe cylinder is loaded from a tube of sterile medical paste in accordance with the manufacturer's instructions.

The Karl Storz device, along with the other prior art devices, lack the ability for consistent and precise injection coupled with one hand operation and ease of replacement of sterile medical paste.

## SUMMARY OF THE INVENTION

The present invention overcomes the disadvantages of the prior art in providing a disposable syringe cylinder which is preloaded with sterile medical paste. The disposable syringe cylinder is similar to that commonly found in the art, except that it contains no thumb knob or dispensing rod. The outer cylinder is filled with the medical paste, and a flexible piston is positioned in the proximal end. The flexible piston provides separation of the sterile medical paste from the external environment. The flexible piston requires no engagement means at its proximal surface.

The present invention further provides for an injector adapted for use with such a disposable syringe cylinder. The syringe is easily inserted into and removed from the injector. The flexible piston of the syringe is employed to propel the medical paste from the syringe and into the catheter. Utilization of the flexible piston in this way preserves the sterile field with a readily disposable element. Except for the disposable syringe cylinder and flexible piston, the remainder of the injector is metal and sterilizable in an autoclave.

The injector provides for pistol grip operation for single hand use. It employs a highly graduated and accurate ratchet for precise dispensing of the desired volume. The ratchet gives aural feedback to indicate the dispensed volume. A special stop rod limits the travel of the pistol grip such that a small (e.g. 0.2 cm$^3$) volume is precisely delivered with each manual stroke.

The distal end of the syringe is coupled to the proximal end of the catheter with a standard Luer coupling. The catheter is of an appropriate length and diameter for use in the urethra. The catheter must have sufficient strength to accommodate the relatively high pressures associated with dispensing of the semi-plastic material.

## BRIEF DESCRIPTION OF THE DRAWINGS

Other objects of the present invention and many of the attendant advantages of the present invention will be readily appreciated as the same becomes better understood by reference to the following detailed description when considered in connection with the accompanying drawings, in which like reference numerals designate like parts throughout the figures thereof and wherein:

Fig. 1     is a plan view of the components of the prior art system;

Fig. 2     is an exploded view of the prior art syringe cylinder;

Fig. 3     is an operational view of the prior art method of filling the syringe cylinder;

Fig. 4     is a sectioned view of the completely filled prior art syringe cylinder;

Fig. 5     is a plan view of a syringe cylinder employing the present invention;

Fig. 6     is a top view of a medical paste injector employing a syringe cylinder according to the present invention;

Fig. 7     is a side view of the main body of an injector incorporating the present invention and adapted for use with a syringe cylinder employing the present invention;

Fig. 8     is a front end view of the injector assembly;

Fig. 9 is an enlarged view of the distal portion of the arrangement of Fig. 6 including the medical paste injector and syringe cylinder according to the present invention;

Fig. 10 is a side view of the injector cutaway showing the stop rod;

Fig. 11 is a view of an alternative embodiment of the injector;

Fig. 12 is a top view of the injector assembly of Fig. 11 after insertion of a prior art syringe cylinder; and

Fig. 13 is a close-up view of the injector and syringe cylinder of Fig. 12 with the disposable syringe cylinder being illustrated in partial phantom showing the dispensing operation.

## DETAILED DESCRIPTION OF THE PREFERRED EMBODIMENT

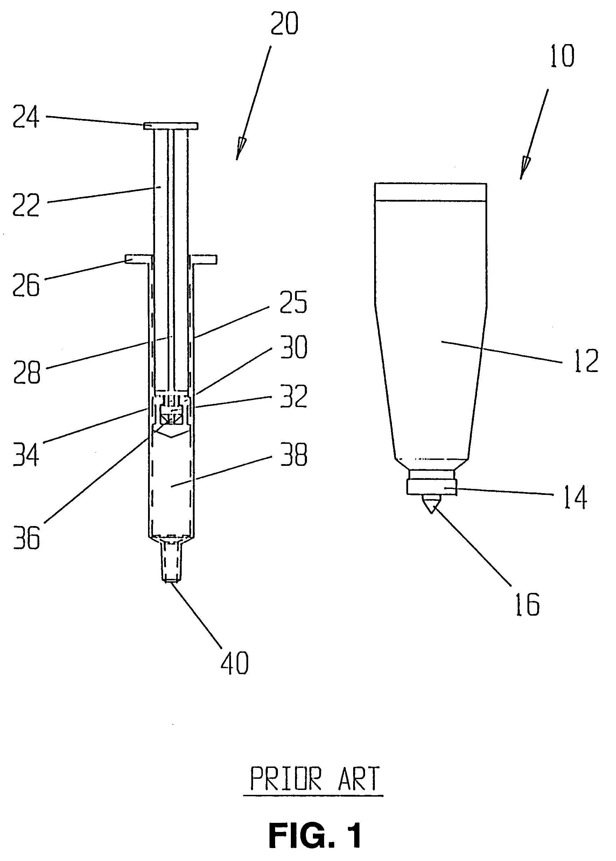

Fig. 1 is a plan view of the components of the prior art system. Tube 10 contains the sterile medical paste to be injected using an injection tool. Typically this may be a PTFE paste available from the assignee of the present invention. Tube 10 contains standard flexible envelope 12 having distal connector 14 and evacuation port 16.

Syringe 20 is a standard disposable syringe. It typically has a main cylinder 25 of a polymer. Interior bore 38 has a constant diameter from proximal to distal end except for distal port 40. Ordinarily distal port 40 is coupled using a standard Luer coupling to a hollow stainless steel needle (not shown). When used to dispense medical paste, distal port 40 is typically coupled to a catheter for remote placement.

Flexible piston 30 is typically an element of generally cylindrical shape of soft rubber. The diameter of flexible piston 30 is chosen to snugly fit interior bore 38. At the proximal end of flexible piston 30 is located a central indentation 32. It is structured to have a smaller proximal bore and a relatively larger more distal bore. However, central indentation 32 does not extend through the distal surface of flexible piston 30.

Central indentation 32 of flexible piston 30 is press fit on to coupling point 36 of evacuation rod 22. This permits evacuation rod 22 to move flexible piston 30 proximally and distally within interior bore 38. Evacuation rod 22 has a generally cruciform shape for strength having rib 28 and three other similar ribs. Thumb knob 24 is appended to the proximal end of evacuation rod 22 for ease of use by the operator. Wings 26 provide for ease in grasping the proximal end of syringe 20.

In normal use, interior bore 38 is vacuum filled with a liquid by manually pulling thumb know 24 and hence flexible piston 30 proximally. The liquid is then dispensed by pushing thumb knob 24 distally. The index and middle finger of the operator grasp wings 26 to permit ease in exerting the desired force. However, the medical paste to be dispensed is too thick to be dispensed in this manner.

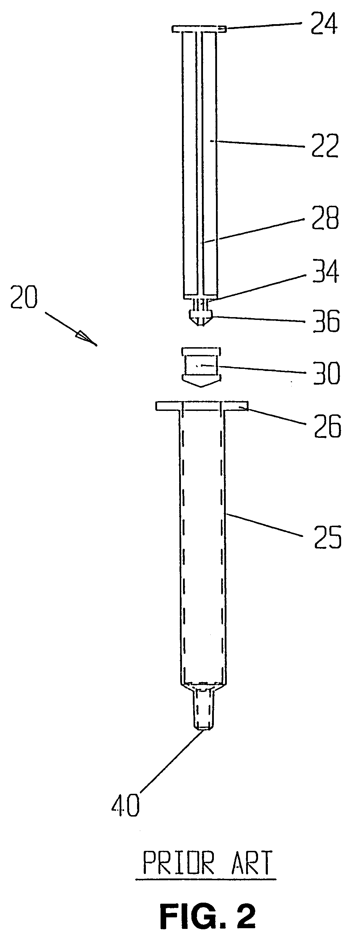

Fig. 2 is an exploded view of prior art syringe 20. All referenced elements are as previously described.

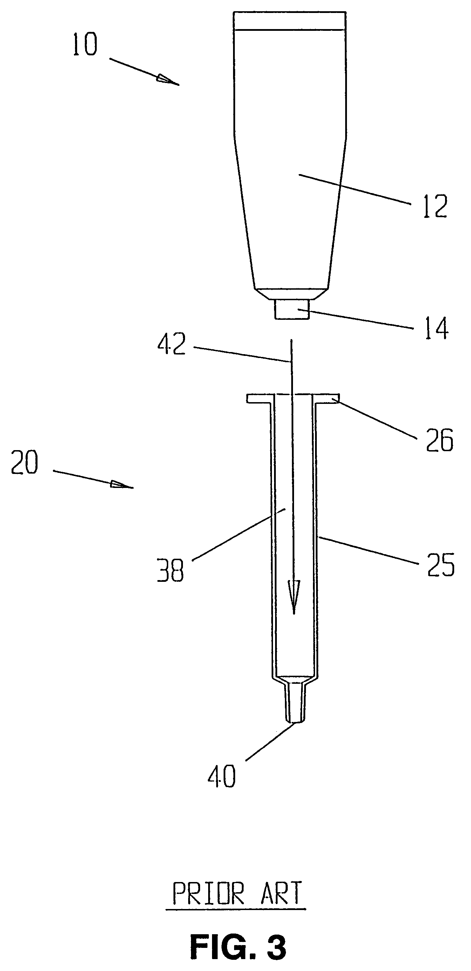

Fig. 3 is a view of the prior art method of filling syringe 20 with medical paste from tube 10. After flexible piston 30 and evacuation rod 22 are completely removed (see also Fig. 2), distal connector 14 is attached to the proximal end of syringe 20 and intermediate wings 26. The medical paste is squeezed from flexible envelope 12 and into interior bore 38 in the direction of arrow 42. All other elements are as previously descriped.

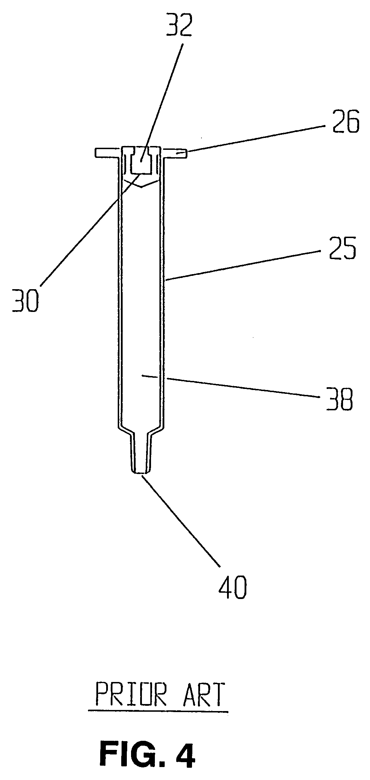

Fig. 4 is a sectioned view of the prior art syringe cylinder as completely filled. After tube 10 is emptied (see also Fig. 3), flexible piston 30 is reinserted to seal the proximal end of interior bore 38. The filled syringe cylinder may now be inserted into an injector tool such as is described in more detail below, or in Karl Storz Model 27200 A medical paste injector. Such tools are required to multiply the manual force to overcome the resistance of the thick medical paste.

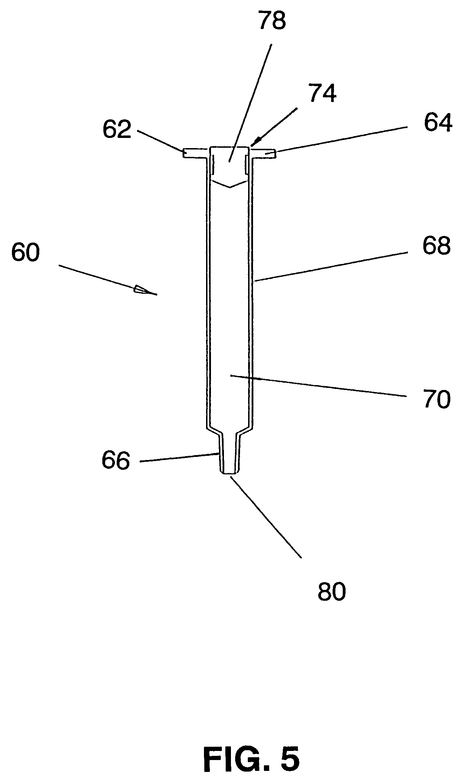

Fig. 5 is a sectioned view of a syringe cylinder 60 according to the present invention. Cylinder body 68 is made of a disposable polymer. Except for distal port 80 at distal end 66 suitable for a standard Luer coupling, it has a substantially constant diameter along its interior bore or lumen 70. Wing tabs 62 and 64 are located at the proximal end of cylinder body 68.

Interior bore 70 is provided completely filled with medical adhesive by the supplier. The proximal end of interior bore 70 including proximal orifice 74 is sealed with flexible piston 78 which is similar to flexible piston 30 except that it preferably has a flat proximal surface. This flat proximal surface permits flexible piston 78 to be moved distally by force supplied from a similarly flat surface. Embodiments are envisioned with proximal surfaces which ar not flat, but also have no means to engage an evacuation rod for pulling flexible piston 78 in a proximal direction. This is important to provide for rapid change of disposable syringe cylinder 60.

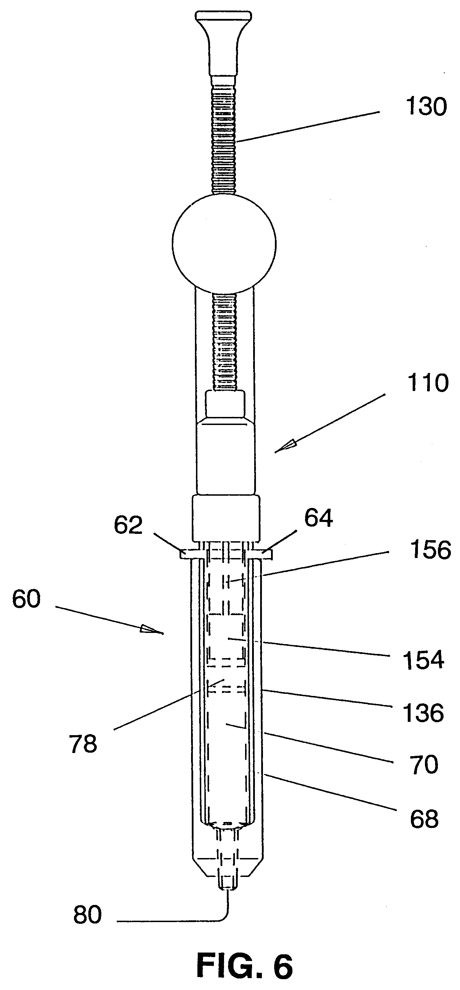

Fig. 6 is a top view of the syringe cylinder 60 of the present invention shown inserted into semi-cylinder 136 of medical paste injector 110 according to the present invention. In operation, medical adhesive is dispensed from distal port 80 by the force applied to flexible piston 78 from rigid cylinder 154 which is attached via rod 156 to rod 130. As rod 130 is advanced, medical paste is dispensed from distal port 80.

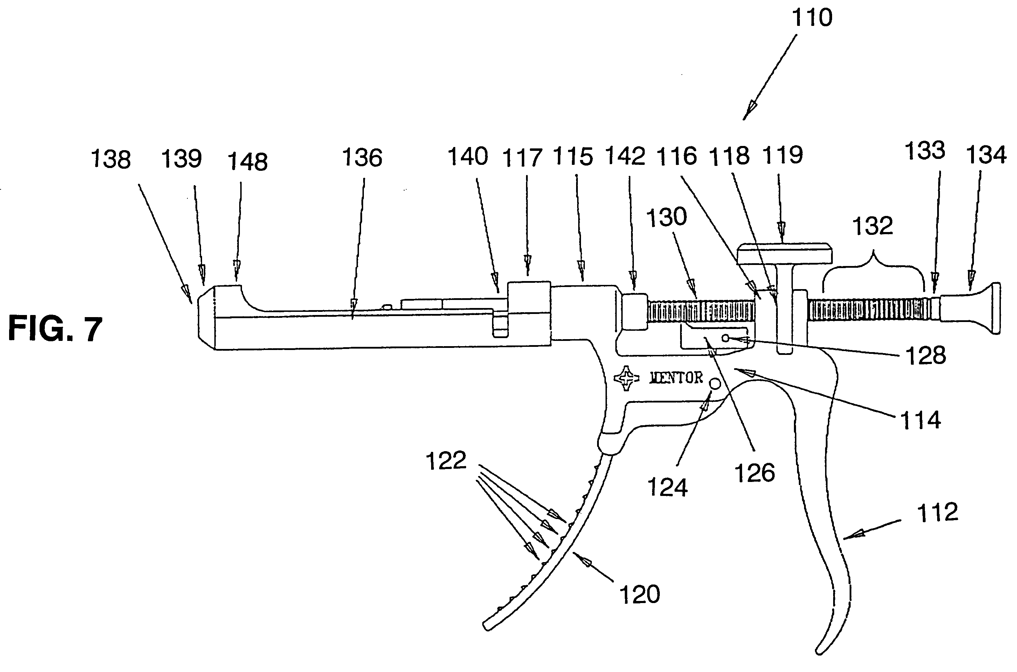

Fig. 7 is a side view of the medical paste injector 110 employing the present invention. The main body 114 of injector 110 is cast of an autoclavable metal having fixed handle 112 configured to fit into the palm of the user's hand. A movable handle 120 having surface ridges 122 is configured to fit the user's fingers. Movable handle 120 pivots at axis 124. The medical paste is propelled by the work performed by the user as movable handle 120 is pulled toward fixed handle 112 as is discussed in further detail below.

Pawl 126 is rotatably coupled to movable handle 120 at axis 128. For this reason, as the user pulls movable handle 120 at surface ridges 122 toward fixed handle 112, movable handle 120 pivots at axis 124 and pushes pawl 126 in the opposite direction (i.e. away from fixed handle 112). The forward edge of pawl 126 engages in ratchet edges 132 of ratchet rod 130 to propel rod 130 toward the left of the drawing. Ratchet engagement latch 118 is slidably held in jig 116. As rod 130 is advanced, latch 118 engages successive ones of ratchet edges 132 preventing movement of rod 130 to the right. In this way, rod 130 is advanced through rod bearing 142.

Rod 130 has knob 134 as its proximal end. Knob 134 is attached via threads (not shown) to stop ring 133. Knob 134 performs two separate functions. It is of sufficient diameter as to prevent rod 130 from advancing to the left any further than the engagement of knob 134 with jig 116. Knob 134 also serves as a convenient place to grasp rod 130. This grasping permits the user to manually pull rod 130 to the right, if button 119 attached to latch 118 is simultaneously depressed manually. This operation resets injector 110 for the insertion of a syringe cylinder as explained in further detail below.

The syringe cylinder (not shown) is inserted into semi-cylinder 136, which is open at the top. The distal end of the syringe cylinder is inserted into cylindrical holder 148. The distal tip of the syringe cylinder exits via coupling 138 which is tapered at incline 139 from cylindrical holder 148. The wing tabs of the syringe cylinder are inserted into slot 140, thus securing the syringe cylinder. The medical paste is propelled by the leftward movement of rod 130 through rod bearing 142 and cylinder housing 117.

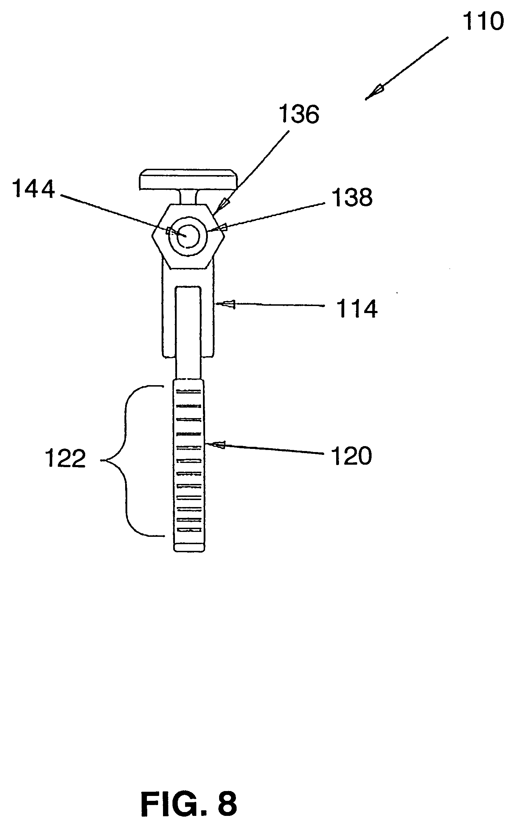

Fig. 8 is a front view of injector 110. Orifice 144 is the interior lumen of coupling 138. All other referenced elements are as previously described.

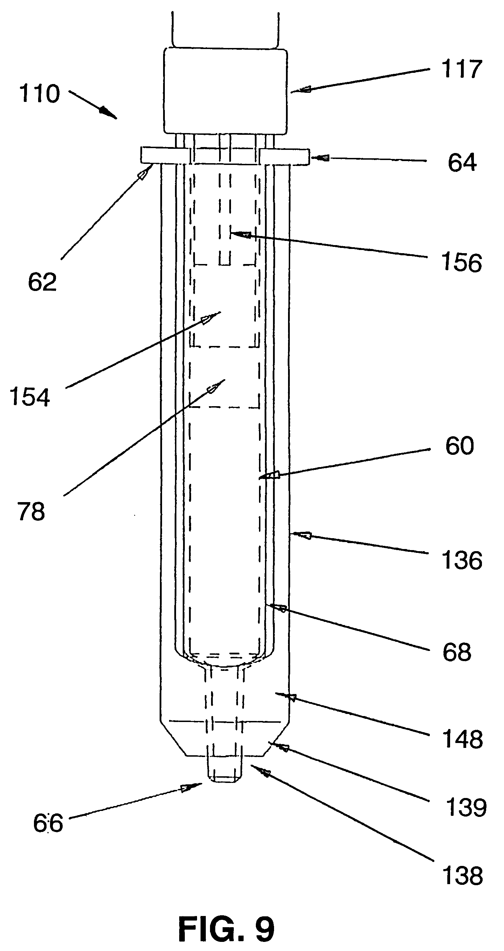

Fig. 9 is a close-up top view of syringe cylinder 60 as positioned within injector 110. Syringe cylinder 60 is supplied as prefilled with flexible piston 78 properly positioned. Rod 156 is the distal extension of rod 130. As can be seen in the drawing, rod 156 is smooth and has no ratchet edges 132. At the distal end of rod 156 is located cylinder 154. Cylinder 154 pushes on the flexible piston 78 of the syringe cylinder 60. Because flexible piston 78 need only move in a single direction (i.e. distally) during the medical procedure, no piston grip nor corresponding slot in flexible piston 78 is required. All other referenced elements are as previously described.

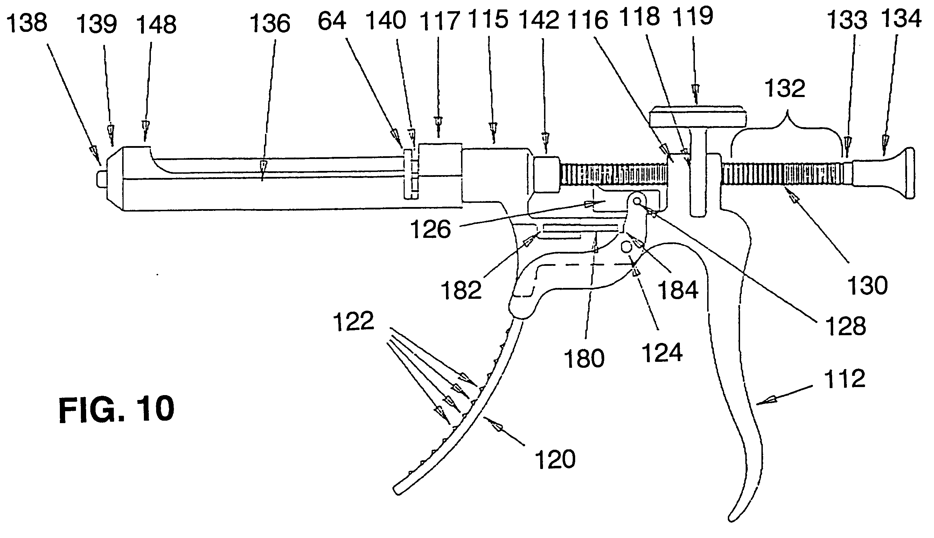

Fig. 10 is a side view of injector 110 showing a partial cut-away of main body 114. Travel stop pin 180 is fixedly mounted in travel stop pin seat 182. It is the function of travel stop pin 180 to stop the travel of movable handle 120 at point 184 as shown. Limiting the travel of movable handle 120 at this precise point 184 limits the volume to be dispensed to a known small amount. In one preferred embodiment, this corresponds to 0.2 cm$^3$.

Aural feedback is also provided to the user by coordinating the specific number of ratchet edges 132 passing latch 118 before travel stop pin 180 contacts point 184. In the preferred embodiment, this is precisely three. Therefore, the user hears three clicks of latch 118 acknowledging the maximum travel of one pull on movable handle 120 and the delivery of 0.2 cm$^3$ of medical paste.

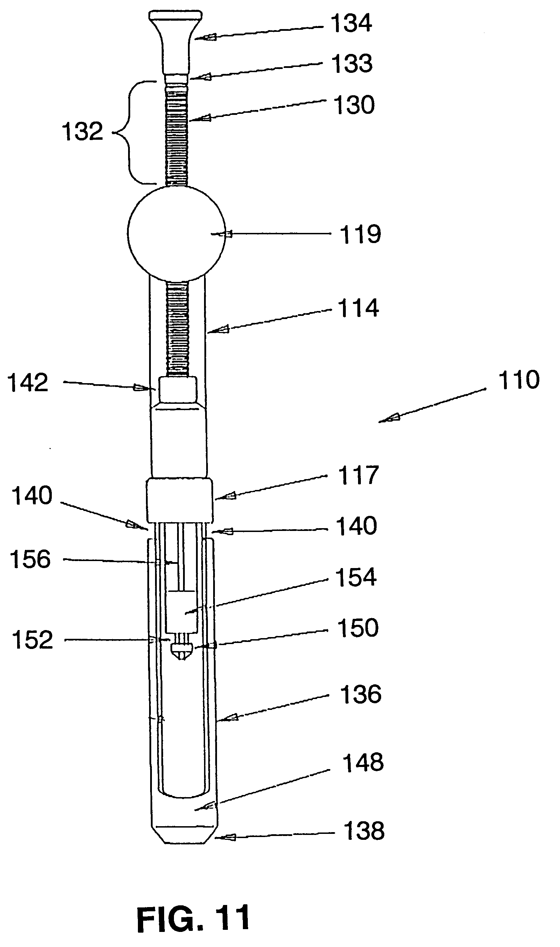

Fig. 11 is top view of an embodiment of injector 110 adapted for use with syringe cylinder 25 of standard syringe 20 shown in Figs. 1 to 3. In this embodiment rod 152 having piston grip 150 is located at the distal end of cylinder 154. Piston grip 150 is adapted to engage the flexible piston 30 as explained in more detail below. All other referenced elements are as previously described.

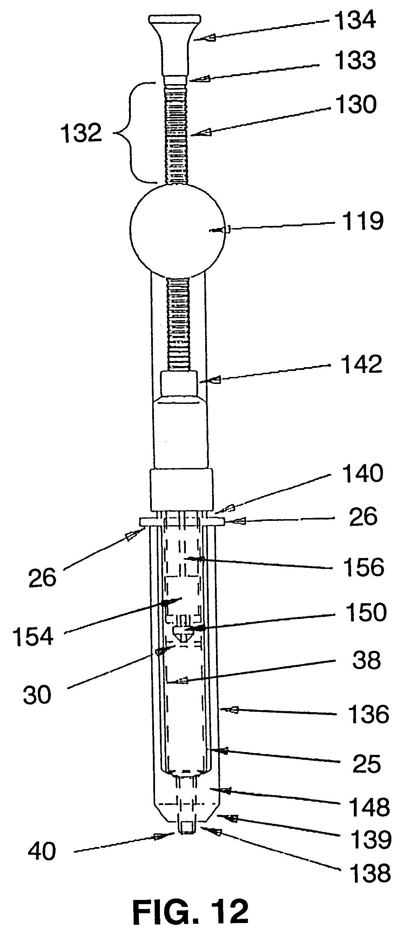

Fig. 12 is a top view of injector 110 shown partially in phantom with syringe cylinder 25 in place. The distal end of cylinder 25 is inserted into coupling 138. The cylinder body is inserted into semi-cylinder 136. Wings 26 are inserted into slot 140. Flexible piston 30 slides downwardly to propel the medical paste from inner lumen 38 as cylinder 154 pushes on flexible piston 30 through the proximal orifice of cylinder 25. Piston grip 150 is engaged into indentation or slot 32 of flexible piston 30.

Syringe cylinder 25 is filled by the user with the medicla paste. To do so, flexible piston 30 and the thumb actuator and rod 22, 24 are removed and inner lumen 38 is filled from a tube of sterile medical paste. The thumb actuator 24 and rod 22 (Fig. 2) are discarded and flexible piston 30 is replaced through the proximal orifice of cylinder 25 to occlude the proximal end of inner lumen 38. All referenced elements are as previously described.

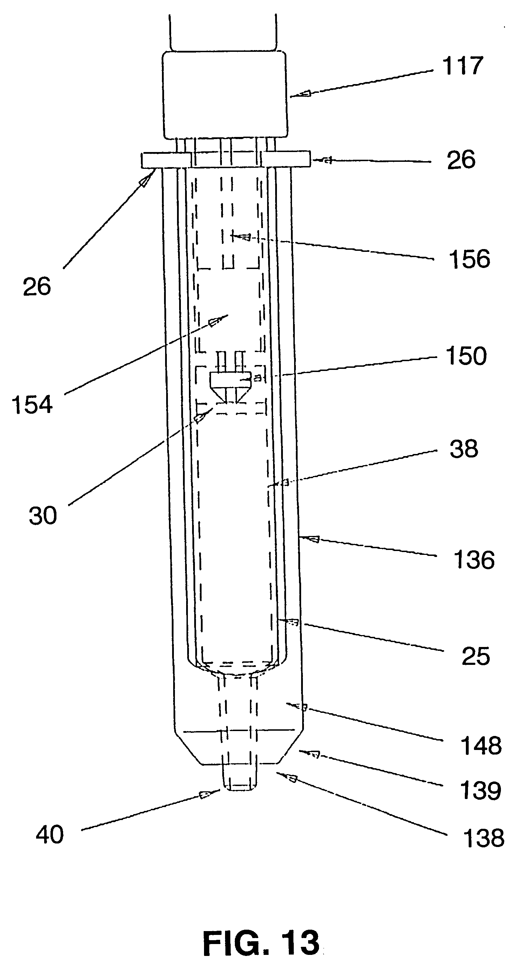

Fig. 13 is a close-up top view of syringe cylinder 25 within injector 110 as partially emptied. All referenced elements are as previously described.

Having thus described the preferred embodiments of the present invention, those of skill in the art will readily appreciate the many other embodiments which can be employed within the scope of the claims hereto attached.

**Claims**

1. An apparatus comprising:
   (a) a syringe cylinder (60) having a substantially constant interior diameter;
   (b) a distal port (74) at a distal end of said syringe cylinder;
   (c) a medical paste filling said syringe cylinder; and
   (d) a flexible piston (72) having a substantially flat proximal surface frictionally engaged within said substantial constant interior diameter.

2. A method of supplying a medical paste comprising:
   (a) filling a syringe cylinder (60) with said medical paste; and
   (b) securing a proximal end of said syringe cylinder with a flexible piston (72) having a substantially flat proximal surface.

3. A medical paste injector (110) comprising:
   (a) a main body (114);
   (b) a fixed handle (112) attached to said main body;
   (c) a movable handle (120) rotatably attached to said main body (114);
   (d) means (60) removably attached to said main body (114) for holding said medical paste;
   (e) means (126, 130, 154, 156) attached to said movable handle (120) and said main body (114) such that movement of said movable handle (120) relative to said fixed handle (112) dispenses said medical paste from said holding means (60); and
   (f) means (180, 182) attached to said main body (114) for limiting said dispensing of said medical paste to a fixed volume.

4. An injector according to claim 3 further comprising means (118, 132) coupled to said main body (114) for providing an aural indication of said fixed volume.

PRIOR ART

# FIG. 1

PRIOR ART

FIG. 2

PRIOR ART

**FIG. 3**

32

26

30

25

38

40

PRIOR ART

# FIG. 4

**FIG. 5**

130

110

62    64

156

60

154

78

136

70

68

80

**FIG. 6**

**FIG. 7**

EP 0 474 218 A1

FIG. 8

**FIG. 9**

FIG. 10

**FIG. 11**

**FIG. 12**

**FIG. 13**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 710 172 (JACKLICH ET AL.) | 1,3,4 | A61M5/315 |
| A | * abstract; figures 2,3 * | 2 | |
| | * column 2, line 18 - line 55 * | | |
| | --- | | |
| X,D | US-A-4 551 135 (GORMAN ET AL.) | 2 | |
| | * abstract; figures 1-4 * | | |
| | * column 3, line 27 - line 46 * | | |
| | --- | | |
| X | US-A-3 517 668 (BRICKSON) | 1,3,4 | |
| A | * column 3, line 24 - line 44 * | 2 | |
| | * column 4, line 54 - column 5, line 3; figure 1 * | | |
| | --- | | |
| X | GB-A-2 142 245 (ORAGAN) | 1-4 | |
| | * abstract; claim 1; figures 1,6,13 * | | |
| | * page 3, line 53 - line 93 * | | |
| | * page 4, line 73 - line 89 * | | |
| | * page 10, line 37 - line 39 * | | |
| | --- | | |
| A | FR-A-2 630 330 (HOEPFNER) | 1-4 | |
| | * abstract; claims 1-3; figures * | | TECHNICAL FIELDS SEARCHED (Int. Cl.5) |
| | --- | | |
| A | WO-A-8 800 843 (MATE ET AL.) | 4 | A61M |
| | * abstract; figures * | | A61C |
| | ----- | | |

The present search report has been drawn up for all claims

| Place of search | Date of completion of the search | Examiner |
|---|---|---|
| THE HAGUE | 28 OCTOBER 1991 | ZEINSTRA H. |

EPO FORM 1503 03.82 (P0401)